(19) Europäisches Patentamt
European Patent Office
Office européen des brevets

(11) Veröffentlichungsnummer : **0 366 977 B1**

# (12) EUROPÄISCHE PATENTSCHRIFT

(45) Veröffentlichungstag der Patentschrift : **24.03.93 Patentblatt 93/12**

(51) Int. Cl.$^5$ : **A61K 6/083,** A61K 6/10

(21) Anmeldenummer : **89118707.2**

(22) Anmeldetag : **09.10.89**

## (54) Mit sichtbarem Licht aushärtbare Dentalmassen.

(30) Priorität : **04.11.88 DE 3837569**

(43) Veröffentlichungstag der Anmeldung : **09.05.90 Patentblatt 90/19**

(45) Bekanntmachung des Hinweises auf die Patenterteilung : **24.03.93 Patentblatt 93/12**

(84) Benannte Vertragsstaaten : **AT BE CH DE ES FR GB IT LI NL SE**

(56) Entgegenhaltungen :
**EP-A- 0 173 567**
**DE-A- 2 157 721**
**DE-A- 3 532 997**
**US-A- 3 912 758**
**WPIL FILE SUPPLIER, AN=89-073385 [10], Derwent Publications Ltd, London, GB; & JP-A-1 026 505 (SHOWA DENKO)**

(73) Patentinhaber : **THERA Patent GmbH & Co. KG**
**Gesellschaft für industrielle Schutzrechte**
**Am Griesberg 2**
**W-8031 Seefeld 1 (DE)**

(72) Erfinder : **Jochum, Peter, Dr.**
**Pointweg 5**
**W-8031 Seefeld 2 (DE)**
Erfinder : **Gasser, Oswald, Dr.**
**Höhenstrasse 10**
**W-8031 Seefeld (DE)**
Erfinder : **Wanek, Erich, Dr.**
**An der Breite 9**
**W-8031 Seefeld (DE)**
Erfinder : **Guggenberger, Rainer, Dr.**
**Inninger Strasse 13a**
**W-8031 Hechendorf (DE)**
Erfinder : **Ellrich, Klaus, Dr.**
**Auinger Strasse 16**
**W-8031 Wörthsee (DE)**

(74) Vertreter : **Bunke, Holger, Dr.rer.nat.**
**Dipl.-Chem. et al**
**Patentanwälte Prinz, Leiser, Bunke & Partner**
**Manzingerweg 7**
**W-8000 München 60 (DE)**

EP 0 366 977 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft photopolymerisierbare, mit sichtbarem Licht aushärtbare Dentalmassen, die - neben üblichen Hilfsund Zusatzstoffen - polymerisierbare Monomere aus der Gruppe der Poly-thiol-Verbindungen mit jeweils wenigstens zwei Thiol-Gruppen und polymerisierbare Monomere aus der Gruppe der Poly-en-Verbindungen mit jeweils wenigstens zwei ethylenisch ungesättigten Gruppen sowie mindestens einen Photoinitiator enthalten.

Es sind bereits eine Reihe von photopolymerisierbaren Massen bekannt, die mit sichtbarem Licht, d.h. mit Licht einer Wellenlänge im Bereich von 400 bis 800 nm, ausgehärtet werden können. So beschreiben die EP-A-0 007 508 und 0 057 474 photopolymerisierbare Massen, enthaltend bestimmte Monoacylphosphinoxide als Photoinitiatoren. Die dort beschriebenen Massen lassen sich mit Licht > 400 nm aushärten; geringe Aushärtungstiefen und Aushärtungsgeschwindigkeiten, noch nicht befriedigende Lagerstabilitäten sowie eine deutliche Schmierschicht, hervorgerufen durch Inhibierung mit Luftsauerstoff, sind jedoch Nachteile, die die Handhabung der dort beschriebenen Massen erschweren und ihre Verwendungsmöglichkeiten begrenzen.

In der DE-OS 34 43 221 sind photopolymerisierbare Massen beschrieben, die Bisacylphosphinoxide enthalten. Diese Massen, die mit sichtbarem Licht gut aushärtbar sind, weisen zwar gegenüber den vorgenannten Massen höhere Aushärtungstiefen und Aushärtungsgeschwindigkeiten sowie bessere Lagerstabilitäten auf, doch ist ihre Oberfläche infolge Inhibierung durch Luftsauerstoff ebenfalls mit einer nachteiligen Schmierschicht überzogen.

Photopolymerisierbare Massen auf Basis Poly-en/Poly-thiol sind beispielsweise aus der US-PS 3 661 744, der DE-OS 35 46 019 sowie der EP-A-0 069 069 bekannt. Sie enthalten neben den Poly-enen und Poly-thiolen UV-Initiatoren wie beispielsweise Benzophenon. Die dort beschriebenen Massen sind jedoch nicht mit sichtbarem Licht einer Wellenlänge > 400 nm aushärtbar.

In der US-PS 3 729 404 sind photohärtbare Poly-en/Poly-thiol-Massen beschrieben, denen als Aktivatoren bestimmte Phosphine zugesetzt sind. Der Zusatz der Phosphine bewirkt eine deutliche Erhöhung der Reaktionsgeschwindigkeit. Eine Härtung mit sichtbarem Licht ist nicht beschrieben.

Aus EP-A-0 173 567 sind photopolymerisierbare, mit sichtbarem Licht härtbare Dentalzusammensetzungen bekannt, bei denen Acylphosphinoxide als Photoinitiatoren verwendet werden. Ein bevorzugt verwendeter Photoinitiator ist 2,4,6-Trimethylbenzoyl-diphenylphosphinoxid. Als polymerisierbare Monomere werden olefinisch ungesättigte Monomere wie z.B. Acrylate und Methacrylate, gegebenenfalls zusammen mit vernetzenden Monomeren, eingesetzt. Die Verwendung von Poly-thiol-Verbindungen ist nicht beschrieben.

Zur Vermeidung der durch die Sauerstoffinhibition hervorgerufenen Schmierschicht bei photopolymerisierbaren Massen wurden bereits eine ganze Reihe von Gegenmaßnahmen vorgeschlagen: Eine Übersicht findet sich in dem Artikel von George F. Vesley "Mechanisms of the Photodecomposition of Initiators" im Journal of Radiation Curing, Januar 1986, Seite 9 - 10. Neben dem technisch sehr aufwendigen Vorschlag, die Aushärtung unter Schutzgasatmosphäre durchzuführen, wird in der Regel vorgeschlagen, hohe Initiatorkonzentrationen einzusetzen oder sogenannte "Sauerstoffänger" den polymerisierbaren Massen zuzusetzen. Als "Sauerstoffänger" eignen sich z.B. tertiäre Amine sowie bestimmte Furane. Hiermit läßt sich eine deutliche Reduktion der Schmierschicht, aber lediglich bei Härtung mit sehr energiereichem Licht kleiner Wellenlänge ( < 400 nm) erzielen. Weiterhin wird vorgeschlagen, oberflächenaktive Photoinitiatoren einzusetzen, mit denen man an der Oberfläche eine deutliche Erhöhung der Photoinitiatorkonzentration und somit eine Verringerung der Schmierschicht erzielen kann. Alle hier beschriebenen Maßnahmen sind bis jetzt jedoch nur mit UV-Initiatoren möglich, die auf Licht einer Wellenlänge < 400 nm ansprechen, wobei eine vollständige Vermeidung der Schmierschicht mit diesen Maßnahmen nicht möglich ist. Durch die Erhöhung der Photoinitiatorkonzentration kommt es bei den ausgehärteten Massen bei weiterer Bestrahlung, z.B. durch Sonnenlicht, außerdem zu verstärkter Verfärbung sowie Zerstörung der Polymermatrix durch Photodegradation, eingeleitet durch nicht verbrauchte Photoinitiatormoleküle.

Alle Versuche, die oben beschriebenen Maßnahmen, die zu einer Verringerung der Schmierschicht bei UV-härtenden Massen beitragen, auf das Gebiet solcher Massen zu übertragen, die mit sichtbarem Licht ( > 400 nm) ausgehärtet werden können, blieben bisher erfolglos. So erhält man etwa bei Kombinationen von Photoinitiatoren, die auf sichtbares Licht ansprechen, wie z.B. Campherchinon und Acylphosphinoxide, mit tertiären Aminen photopolymerisierbare Massen, die zwar mit sichtbarem Licht gehärtet werden können, aber nach der Härtung infolge der Sauerstoffinhibition eine deutliche Schmierschicht aufweisen (vgl. DE-OS 34 43 221). Deswegen bedient man sich auf dem Gebiet der Dentalmaterialien relativ aufwendiger Verfahren, um die Bildung einer Schmierschicht zu verhindern. So wurde vorgeschlagen, die Aushärtung von dentalen Verblendmaterialien in einem Flüssigkeitsbad durchzuführen (DE-OS 33 16 591) oder die Aushärtung unter Vakuum durchzuführen (DE-OS 30 01 616).

Der Erfindung liegt daher die Aufgabe zugrunde, neue, mit sichtbarem Licht schmierschichtfrei aushärtbare Massen zu schaffen, die insbesondere als Dentalmassen eingesetzt werden können.

Diese Aufgabe wird durch die Bereitstellung der erfindungsgemäßen Dentalmassen gelöst.

Gegenstand der Erfindung sind photopolymerisierbare, mit sichtbarem Licht aushärtbare Dentalmassen, die - neben üblichen Hilfs- und Zusatzstoffen - polymerisierbare Monomere aus der Gruppe der Poly-thiol-Verbindungen mit jeweils wenigstens zwei Thiol-Gruppen und polymerisierbare Monomere aus der Gruppe der Poly-en-Verbindungen mit jeweils wenigstens zwei ethylenisch ungesättigten Gruppen sowie mindestens einen Photoinitiator enthalten, welche dadurch gekennzeichnet sind, daß die Massen, jeweils bezogen auf die Summe aller polymerisierbarer Monomere,

(a) mindestens 10 Gew.-% einer oder mehrerer der Poly-thiol-Verbindungen,

(b) mindestens 10 Gew.-% einer oder mehrerer der Poly-en-Verbindungen und

(c) als Photoinitiator 0,01 - 5 Gew.-% mindestens einer Acylphosphinverbindung der allgemeinen Formel I enthalten,

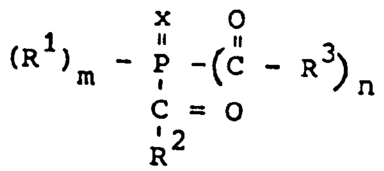

(I)

worin

m = 1, n = 1 und x = O

oder

m = 2, n = 0 und x = O oder S,

$R^1$ einen geradkettigen oder verzweigten $C_{1-6}$-Alkylrest,

einen Cyclopentyl-, Cyclohexyl-, Phenyl-, Naphthyl- oder Biphenylylrest, der gegebenenfalls substituiert ist durch F, Cl, Br, J, $C_1$-$C_{12}$-Alkyl und/oder $C_1$-$C_{11}$-Alkoxyl, einen S- oder N-haltigen 5- oder 6-gliedrigen heterocyclischen Ring;

$R^2$ und $R^3$, die gleich oder verschieden sind,

einen Cyclohexyl-, Cyclopentyl-, Phenyl-, Naphthyl- oder Biphenylylrest,

einen Cyclopentyl-, Cyclohexyl-, Phenyl-, Naphthyl- oder Biphenylylrest, der substituiert ist durch F, Cl, Br, J, $C_1$-$C_{12}$-Alkyl und/oder $C_1$-$C_{11}$-Alkoxyl, oder einen S- oder N-haltigen 5- oder 6-gliedrigen heterocyclischen Ring

bedeuten oder

$R^2$ und $R^3$ miteinander zu einem Ring verknüpft sind, der 4 bis 10 Kohlenstoffatome enthält und durch 1 bis 6 $C_{1-4}$-Alkylreste substituiert sein kann.

Die erfindungsgemäßen Dentalmassen können zusätzlich zu den Komponenten (a) bis (c) als Komponente (d) 20 - 79,99 Gew.-%, bezogen auf die Gesamtheit aller polymerisierbarer Monomere, eines Acrylsäure- und/oder Methacrylsäureesters eines mindestens difunktionellen Alkohols enthalten.

Gegenstand der Erfindung ist ferner die Verwendung der erfindungsgemäßen Dentalmassen als dentale Abformmassen.

Die erfindungsgemäßen Massen lassen sich überraschenderweise mit sichtbarem Licht einer Wellenlänge > 400 nm schmierschichtfrei aushärten. Damit lassen sich erstmals die Vorteile der Härtung mit sichtbarem Licht - hierzu gehören die Erzielung großer Aushärtungstiefen, selbst bei stark pigmentierten Systemen, die Umgehung der Eigenabsorption der Polymermatrix sowie die Möglichkeit, auch durch Substrate, z.B. durch Polycarbonat, PMMA und dergl. hindurch zu bestrahlen - mit dem Vorteil kombinieren, schmierschichtfreie Oberflächen ohne besonderen apparativen Aufwand wie z.B. Vakuum, Schutzgas oder Flüssigkeitsbad zu erhalten. Weitere Vorteile der erfindungsgemäßen Massen sind hohe Lagerstabilität, große Aushärtungsgeschwindigkeit, geringe Vergilbungstendenz sowie geringer Restmonomergehalt.

Den erfindungsgemäßen Massen eröffnen sich somit zahlreiche vorteilhafte Einsatzmöglichkeiten. Genannt seien beispielsweise Beschichtungen, Einbettungen, Vergüsse und Verklebungen innerhalb und außerhalb des Dentalgebietes sowie generell das Aushärten aller Massen, bei denen nach der Härtung freie Oberflächen zugänglich sind.

So können die erfindungsgemäßen Massen beispielsweise eingesetzt werden, um aus Metall gefertigte Kronen- und Brückenteile mit zahnähnlich eingefärbten und mit sichtbarem Licht aushärtbaren Dentalmassen

zu beschichten. Hierbei entfällt das bisher nötige aufwendige Verarbeiten unter Vakuum oder Flüssigkeit. Ganz besonders vorteilhaft lassen sich die Eigenschaften der erfindungsgemäßen Massen bei der Abdrucknahme im Munde eines Patienten ausnützen. Neben der bisher bekannten Anwendung von zweikomponentigen Massen, die vor der Abdrucknahme erst homogen vermischt werden müssen, waren bisher lediglich lichthärtende Abdruckmassen auf Basis radikalisch härtender Acrylatsysteme bekannt, vgl. z.B. EP-A- 0 170 219, 0 255 286. Bei diesen Massen besteht aber aufgrund der Inhibierung durch Luftsauerstoff an allen freien und feuchten Oberflächen die Gefahr der Bildung einer verbleibenden deutlichen Schmierschicht, die für den Zahnarzt eine extreme Verschmutzungsgefahr in sich birgt, da diese Oberflächen auch stark klebrig sind. Außerdem sind mit diesen Massen genommene Abdrücke für den Zahntechniker sehr schwierig auszugießen, da sich die Masse an den nicht polymerisierten Teilen der Oberfläche mit dem Modellmaterial vermischen kann.

Mit den erfindungsgemäßen Massen lassen sich diese Nachteile vermeiden. Die für die Abdrucknahme erforderlichen Elastomereneigenschaften lassen sich durch entsprechende Auswahl der Poly-thiol- und Polyen-Komponenten sowie der eingesetzten Füllstoffe in üblicher Weise einstellen.

Die erfindungsgemäß einzusetzenden Poly-thiole der Komponente (a) werden vorzugsweise in einer Menge von mindestens 20 Gew.-%, bezogen auf polymerisierbare Monomere, verwendet. Vorzugsweise ist mindestens ein Drittel der eingesetzten Poly-thiol-Verbindungen wenigstens trifunktionell, enthält also wenigstens drei Thiolgruppen pro Molekül. Brauchbare Poly-thiol-Verbindungen sind beschrieben in den US-PS 3 661 744 und 4 119 617. Die eingesetzten Poly-thiol-Verbindungen haben zweckmäßigerweise ein Molekulargewicht im Bereich zwischen 150 und 20.000; vorteilhaft ist ein Molekulargewicht von mindestens 500, besonders bevorzugt von mindestens 1000.

Für die Verwendung zur Herstellung von mit sichtbarem Licht schmierschichtfrei aushärtbaren Abformmassen werden vorteilhaft Poly-thiol-Verbindungen mit einem mittleren Molekulargewicht von 500 - 10.000, besonders bevorzugt von 1000 bis 7000, eingesetzt.

Als Poly-thiol-Verbindungen können erfindungsgemäß Verbindungen verwendet werden, die der allgemeinen Formel

$$R^0 - (SH)_n$$

entsprechen, worin $R^0$ für einen organischen Rest steht, der vorzugsweise frei ist von ethylenisch ungesättigten Doppelbindungen, und n eine ganze Zahl $\geqq 2$, vorzugsweise 2, 3 oder 4, bedeutet.

Gut geeignete Poly-thiol-Verbindungen sind beispielsweise Polyoxyalkylentriole mit Mercaptan-Endgruppen. Beispiele für solche Verbindungen sind das im Handel erhältliche Capcure® 3-800 mit der folgenden Formel

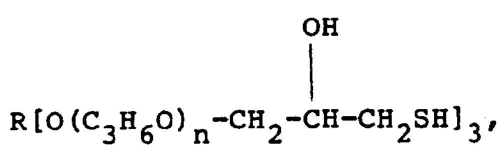

worin R einen aliphatischen Kohlenwasserstoffrest mit 1 - 12C-Atomen und n eine ganze Zahl von 1 bis 25 bedeuten, sowie Capcure ® 5-1300 mit der folgenden Formel

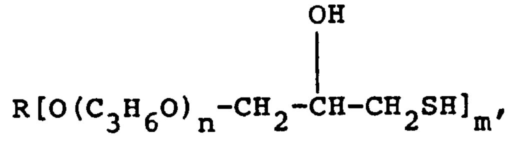

worin R einen aliphatischen Kohlenwasserstoffrest mit 1 - 12C-Atomen, n die Zahl 1 oder 2 und m die Zahl 5 oder 6 bedeuten.

Für die Verwendung als Abformmassen gut geeignete Poly-thiol-Verbindungen sind auch die Umsetzungsprodukte von Mercaptocarbonsäuren mit Polyoxyalkylen wie beispielsweise Polyethylenoxiden mit mindestens zwei endständigen Hydroxygruppen, Polypropylenoxide bzw. Copolymere aus Ethylenoxid und Propylenoxid, die jeweils mindestens zwei endständige Hydroxygruppen haben. Besonders gut geeignet sind auch Copolymere aus Ethylenoxid mit Tetrahydrofuran. Diese vorgenannten Polyether-di- oder - poly-ole haben vorzugsweise ein mittleres Molekulargewicht von 500 - 10.000, besonders bevorzugt von 1500 bis 7000.

Gut geeignet sind ferner die Ester von Mercaptocarbonsäuren mit mindestens trifunktionellen Alkoholen. Beispiele für diese Verbindungsklasse sind die Mercaptocarbonsäureester des Trimethylolpropans und des Pentaerythrits. Die hierbei verwendeten Mercaptocarbonsäuren haben Kohlenstoffgerüste mit 2 bis 20 C-Atomen, vorzugsweise 5 bis 15 C-Atomen. Gut geeignet sind beispielsweise Trimethylolpropan-trismercaptoundecanoat oder Pentaerythrit-tetrakismercaptoundecanoat. Ein ebenfalls gut geeignetes Poly-thiol ist das Tris-mercaptoethylisocyanurat.

Die erfindungsgemäß einzusetzenden Poly-en-Verbindungen werden in einer Menge von mindestens 10 Gew.-%, bezogen auf polymerisierbare Momomere, eingesetzt, wobei mindestens 20 Gew.-% bevorzugt sind. In einer besonders bevorzugten Ausführungsform werden Poly-thiol- und Poly-en-Verbindungen als einzige Monomere eingesetzt, so daß sie zusammen also 100 Gew.-% der polymerisierbaren Monomere ausmachen.

Die wenigstens 2 ethylenisch ungesättigten Gruppen ("en-Gruppen") der erfindungsgemäß einzusetzenden Poly-en-Verbindungen können gegebenenfalls substituiert sein. Vorzugsweise enthält mindestens ein Drittel der eingesetzten Poly-en-Verbindungen mindestens 3 "en-Gruppen", die gegebenenfalls substituiert sein können. Unter en-Gruppen sind definitionsgemäß z.B. zu verstehen: O-Allyl-, N-Allyl-, O-Vinyl-, N-Vinyl- und p-Vinyl-phenolether-Gruppen. Mögliche Poly-ene sind beschrieben in den US-PS 3 661 744 und in der EP-A-0 188 880. Das Poly-en kann z.B. die folgende Struktur aufweisen:

$$(A)\text{-}(X)_m,$$

wobei m eine ganze Zahl $\geqq 2$, vorzugsweise 2, 3 oder 4, ist und X ausgewählt ist aus der Gruppe

(i) 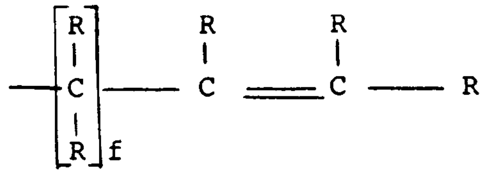

(ii) 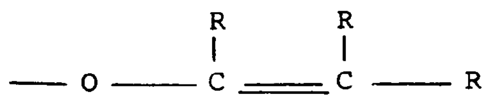

(iii) 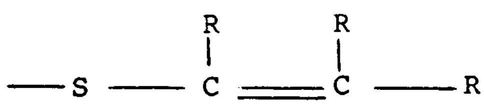

(iv) 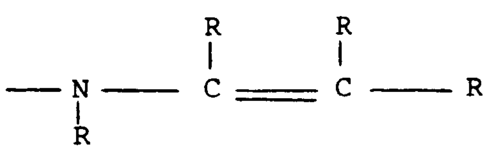

worin f eine ganze Zahl von 1 bis 9 ist und der Rest R die Bedeutungen H, F, Cl, Furyl, Thienyl, Pyridyl, Phenyl und substituiertes Phenyl, Benzyl und substituiertes Benzyl, Alkyl und substituiertes Alkyl, Alkoxy und substituiertes Alkoxy sowie Cycloalkyl und substituiertes Cycloalkyl besitzen und jeweils gleich oder verschieden

sein kann.

(A) ist ein mindestens difunktioneller, organischer Rest, der aus den Atomen aufgebaut ist, die ausgewählt sind aus der Gruppe C, O, N, Cl, Br, F, P, Si und H.

Die Poly-en-Verbindungen haben vorzugsweise mittlere Molekulargewichte im Bereich von 64 - 20.000, vorzugsweise 200 - 10.000 und besonders bevorzugt 500 - 5000.

Gut geeignete Poly-en-Verbindungen sind beispielsweise die Allyl- und/oder Vinylester mindestens difunktioneller Carbonsäuren. Als Carbonsäuren eignen sich hierfür solche mit Kohlenstoffketten von 2 bis 20 C-Atomen, vorzugsweise von 5 bis 15 C-Atomen; gut geeignet sind auch Allyl- und Vinylester aromatischer Dicarbonsäuren wie Phthalsäure oder Trimellithsäure. Beispielhaft genannt seien Bernsteinsäurediallylester sowie Bernsteinsäuredivinylester, Phthalsäurediallylester und Phthalsäuredivinylester.

Gut geeignet sind auch Allylether polyfunktioneller Alkohole, beispielsweise Polyether-di- oder -poly-ole, wie Polyethylenoxide, Polypropylenoxide, deren Copolymere oder auch Copolymere aus Ethylenoxid und Tetrahydrofuran. Bevorzugt sind die Allylether mindestens trifunktioneller Alkohole. Als Beispiel seien genannt die Allylether des Trimethylolpropans, Pentaerythrittriallylether-acrylat oder 2,2-Bis-oxyphenylpropan-bis-(diallyl-phosphat).

Gut geeignet sind auch Verbindungen des Typs Cyanursäure-triallylester, Triallyl-triazin-trion und dergl.

Bei zusätzlicher Anwesenheit der Komponente (d) kann es von Vorteil sein, polyfunktionelle Alkohole teilweise mit Allylether-Endgruppen und teilweise mit Acrylsäureester- bzw. Methacrylsäureester-Endgruppen einzusetzen.

Das Molverhältnis von einzusetzendem Poly-thiol zu Poly-en ist so auszuwählen, daß eine vollständige Polymerisation möglich ist. Hierbei beträgt das Verhältnis von En- zu Thiol-Gruppen normalerweise 0,2 : 1 bis 5 : 1, vorzugsweise 0,75 : 1 bis 1,5 : 1.

Als Abformmassen gut geeignet sind solche, bei denen mindestens eine der Komponenten (Thiol oder En) ein Polyethermittelstück der o.g. Art besitzt.

Die erfindungsgemäß einzusetzenden Acylphosphin-Verbindungen werden vorzugsweise in einer Menge von 0,1 - 3 Gew.-%, besonders bevorzugt von 0,2 bis 1 Gew.-%, jeweils bezogen auf polymerisierbare Monomere, eingesetzt.

Bevorzugte Acylphosphin-Verbindungen der allgemeinen Formel I sind Monoacylphosphinoxide, wobei 2,4,6-Trimethylbenzoyl-diphenylphosphinoxid besonders bevorzugt ist. Vorteilhaft verwendet werden können auch Bisacylphosphinoxide, beispielsweise Bis-(2,6-dichlorbenzoyl)-4′-n-propylphenylphosphinoxid.

Wie erwähnt, können 20 - 79,99 Gew.-% der polymerisierbaren Monomere aus Acrylsäure- und/oder Methacrylsäureestern mindestens difunktioneller Alkohole bestehen. Bevorzugt werden 40 - 60 Gew.-% eingesetzt. Als Acrylsäure- und/oder Methacrylsäureester eingesetzt werden können monomere und polymere Acrylate und Methacrylate. Gut geeignet sind z.B. die langkettigen Monomere gemäß der US-PS 3 066 112 auf der Basis von Bisphenol-A und Glycidylmethacrylat oder deren durch Addition von Isocyanaten entstandene Derivate; geeignet sind auch die Acrylsäure- und Methacrylsäureester ein- oder mehrwertiger Alkohole, beispielsweise Methyl- und Ethylmethacrylat, insbesondere die Ester mehrwertiger Alkohole wie Triethylenglykol-di(meth)acrylat und Trimethylol-propan-tri(meth)acrylate Geeignet sind auch Verbindungen des Typs Bisphenol A-bis-oxy-ethyl(meth)acrylat und Bisphenol A-bis-oxy-propyl(meth)acrylat.

Besonders geeignet sind die in der DE-PS 28 16 823 genannten Diacryl- und Dimethacrylsäureester des Bis-hydroxymethyl-tri-cyclo [5.2.1.$0^{2,6}$]-decans. Verwendet werden können auch die Reaktionsprodukte aus Diisocyanaten und Hydroxyalkyl(meth)acrylaten, wie sie in der DE-OS 23 12 559 beschrieben sind. Selbstverständlich können auch Gemische aus Monomeren und/oder aus hieraus hergestellten ungesättigten Polymeren verwendet werden.

Die erfindungsgemäßen Dentalmassen können ferner übliche Hilfsund Zusatzstoffe, z.B. Füllstoffe, Thixotropiehilfsmittel, Farb- und Aromastoffe sowie Weichmacher, enthalten. Als Füllstoffe geeignet sind alle im Dentalbereich bei mit sichtbarem Licht härtbaren Massen üblicherweise eingesetzten Füllstoffe wie feingemahlenes Glas, Quarzglas, Quarz, Calciumcarbonat und dergl. Die Füllstoffe haben vorzugsweise eine Kornverteilung im Bereich von 0,001 - 50 μm, besonders bevorzugt ist ein Bereich von 0,001 bis 10 μm. Gut einsetzbar sind feinteilige Kieselsäuren wie pyrogene Kieselsäure oder Fällungskieselsäure, die gleichzeitig auch als Thixotropiehilfsmittel wirken können. Die eingesetzten Füllstoffe und Thixotropiehilfsmittel sind vorzugsweise silanisiert. Als Silanisierungsmittel können die an sich bekannten Silankupplungsmittel, beispielsweise γ-Methacryloxypropyltrimethoxysilan verwendet werden. Die Menge an Füllstoffen kann je nach Einsatzgebiet in einem weiten Bereich schwanken. Sie beträgt bis zu 90 Gew.-%, bezogen auf die Gesamtmasse; bevorzugt eingesetzt wird ein Bereich von 10 - 50 Gew.-%.

Als Weichmacher können alle in der Kunststoff- und Kautschukchemie üblicherweise eingesetzten Weichmacher verwendet werden. Gut geeignet sind beispielsweise Copolymere aus Ethylen- und Propylenoxid, Dialkylphthalate, beispielsweise Dioctylphthalat, Dibenzyltoluol oder Acetyltributylcitrat. Die Menge an Weichmachern hängt ebenfalls stark vom Verwendungszweck ab. Für dentale Abformmassen hat sich ein Konzentrationsbereich von 0 - 20 Gew.-%, bevorzugt von 0 - 5 Gew.-%, ais vorteilhaft erwiesen.

Die Erfindung wird nachfolgend anhand der Beispiele weiter erläutert:

Beispiel 1:

(a) 50 Gewichtsteile Pentaerythrit-tetramercaptoproprionat,
(b) 50 Gewichtsteile Triallyltriazintrion und
(c) 0,5 Gewichtsteile 2,4,6-Trimethylbenzoyl-diphenylphosphinoxid

werden mit 0,1 Gewichtsteilen Brenzcatechin (Inhibitor zur Verhinderung der vorzeitigen Polymerisation) zu einer homogenen Lösung vermischt.

Die so erhaltene transparente Lösung wird in eine zylindrische Form aus Polyoxymethylen (Durchmesser 5 mm, Länge 40 mm) eingefüllt. Die Masse wird durch die offene Stirnseite der zylindrischen Form mittels eines handelsüblichen dentalen Bestrahlungsgeräts (Elipar-Visio/ESPE) 20 Sekunden lang mit sichtbarem Licht einer Wellenlänge von > 400 nm belichtet. Zur Bestimmung der Aushärtungstiefe bzw. der Dicke der ausgehärteten Schicht werden die von der bestrahlten Oberfläche abgewandten, weichen oder gelartigen, nicht durchpolymerisierten Bestandteile mit einem Kunststoffspatel entfernt, und die ausgehärtete Schichtdicke wird gemessen. Es zeigte sich, daß mit sichtbarem Licht und bei einer Belichtungsdauer von 20 Sekunden eine ausgehärtete Schichtdicke von 30 mm erreichbar ist, wobei die Oberflächenhärte der ausgehärteten Masse 200 MPa beträgt.

Bei der Durchhärtung eines Materialtropfens zeigte sich, daß der Tropfen bereits nach wenigen Sekunden vollständig durchgehärtet ist und daß die Oberfläche absolut trocken ist, d.h. keine Schmierschicht aufweist.

Die erfindungsgemäße Masse ist somit hervorragend geeignet zur Herstellung von Beschichtungslacken für Dentalgipse, Prothesen und Kronen- und Brückenverblendungsmaterialien. Ebenso hervorragend geeignet ist die erfindungsgemäße Masse aber auch zur Herstellung von Einbettungen, Verklebungen und Beschichtungen von elektrischen und elektronischen Bauteilen.

Beispiel 2:

(a) 12,2 Gewichtsteile Pentaerythrit-tetramercaptopropionat,
(b) 6,2 Gewichtsteile Triallyltriazintrion,
(c) 0,068 Gewichtsteile Bis-(2,6-dichlorbenzoyl)-4-N-propylphenylphosphinoxid und
(d) 6,4 Gewichtsteile 4,4′-Bis-(acryloyloxyethyl-oxy-ethoxy)-2,2-diphenylpropan,

werden bei Raumtemperatur zu einer homogenen Mischung gerührt. Die Mischung hat eine Viskosität von 0,7 Pa.s. Die so erhaltene transparente Lösung wird in eine zylindrische Form aus Polyoxymethylen (Durchmesser 5 mm, Länge 40 mm) eingefüllt. Die Masse wird durch die offene Stirnseite der zylindrischen Form mittels eines handelsüblichen dentalen Bestrahlungsgerätes (Elipar-Visio/Espe) 20 Sekunden lang mit sichtbarem Licht einer Wellenlänge von > 400 nm belichtet. Zur Bestimmung der Aushärtungstiefe bzw. der Dicke der ausgehärteten Schicht werden die von der bestrahlten Oberfläche abgewandten, weichen oder gelartigen, nicht durchpolymerisierten Bestandteile mit einem Kunststoffspatel entfernt, und die ausgehärtete Schichtdicke wird gemessen. Es zeigt sich, daß mit sichtbarem Licht und bei einer Belichtungsdauer von 20 Sekunden eine ausgehärtete Schichtdicke von 15 mm erreichbar ist, wobei die Oberflächenhärte der ausgehärteten Masse 250 MPa beträgt.

Bei der Durchhärtung eines Materialtropfens zeigt sich, daß der Tropfen bereits nach wenigen Sekunden vollständig durchgehärtet ist und die Oberfläche absolut trocken ist, d.h. keine Schmierschicht aufweist. Die erfindungsgemäße Masse ist somit hervorragend geeignet zur Herstellung von Beschichtungslacken für Dentalgipse, Prothesen und Kronen- und Brückenverblendungsmaterialien, aber auch zur Herstellung von Einbettungen, Verklebungen und Beschichtungen von elektrischen und elektronischen Bauelementen.

Beispiel 3:

12 g des Umsetzungsproduktes von Allylchlorid (2 Mol) mit einem Diol (1 Mol), hergestellt aus der Copolymerisation gleicher Gewichtsteile Tetrahydrofuran und Ethylenoxid mit einem mittleren Molekulargewicht von ca. 6000, werden mit 1 Gewichtsteil Trimethylolpropan-trimercaptoundecanoat, 0,03 Gewichtsteilen Brenzcatechin und 0,13 Gewichtsteilen 2,4,6-Trimethylbenzoyl-diphenylphosphinoxid zu einer homogenen klaren Lösung verarbeitet. 40 Gewichtsteile der so hergestellten Lösung werden mit 30 Gewichtsteilen silanisierter pyrogener Kieselsäure mit einer spezifischen Oberfläche von 140 $m^2/g$ zu einer homogenen thixotropen Paste verknetet, die unter Druck fließfähig, im Ruhezustand aber standfest ist.

Ein Tropfen dieses Materials ist nach einer 40 Sekunden dauernden Belichtung mit sichtbarem Licht einer Wellenlänge > 400 nm unter Verwendung eines handelsüblichen dentalen Bestrahlungsgeräts (Elipar-Visio/ESPE) vollständig durchpolymerisiert und das Polymerisat besitzt eine glatte, absolut trockene Oberfläche ohne Schmierschicht. Die so entstandene, ausgehärtete Masse ist gummielastisch bzw. elastomer, verfügt über hervorragende elastische Eigenschaften und ist extrem reißfest. Zur Bestimmung der Shore-A-Härte nach DIN 53 505 wird ein zylindrischer Prüfkörper mit einem Durchmesser von 2,5 cm und einer Höhe von 3,5 mm durch 2-minütiges Bestrahlen mit dem Elipar-Visio-Gerät hergestellt. Die Shore-A-Härte beträgt 52 und verändert sich nach weiterem Bestrahlen nicht mehr.

Ein handelsübliches lichthärtendes Abformmaterial ("Genesis", Hersteller: Caulk) auf Acrylatbasis ergibt nach der gleichen Belichtungsdauer mit dem Elipar-Visio-Gerät einen mäßig elastischen, reißfesten Gummi mit einer Shore-A-Härte von 70. Ideal für die Anwendung als dentale Abformmasse ist eine Shore-A-Härte im Bereich von 50 - 60, da nur dann gewährleistet ist, daß bei der Entnahme aus dem Mund des Patienten keine Schädigung des Zahnmaterials zu befürchten ist und daß beim Ausgießen des Abdrucks mit Gips und anschließendem Entformen die Gipsstümpfe nicht abbrechen. Ein ausgehärteter Tropfen des handelsüblichen Produktes (Vergleichsmaterial) ist an allen freien Oberflächen mit einer abwischbaren Schmierschicht behaftet. Auch nach dem Abwischen der Schmierschicht verbleibt eine klebrige Oberfläche.

Somit ist die erfindungsgemäße Masse als dentale Abformmasse hervorragend geeignet.

## Patentansprüche

1. Photopolymerisierbare, mit sichtbarem Licht aushärtbare Dentalmassen, die - neben üblichen Hilfs- und Zusatzstoffen -polymerisierbare Monomere aus der Gruppe der Poly-thiol-Verbindungen mit jeweils wenigstens zwei Thiol-Gruppen und polymerisierbare Monomere aus der Gruppe der Poly-en-Verbindungen mit jeweils wenigstens zwei ethylenisch ungesättigten Gruppen sowie mindestens einen Photoinitiator enthalten, **dadurch gekennzeichnet**, daß die Massen, jeweils bezogen auf die Summe aller polymerisierbarer Monomere,

   (a) mindestens 10 Gew.% einer oder mehrerer der Poly-thiol-Verbindungen,

   (b) mindestens 10 Gew.% einer oder mehrerer der Poly-en-Verbindungen und

   (c) als Photoinitiator 0,01 bis 5 Gew.% mindestens einer Acylphosphinverbindung der allgemeinen Formel I enthalten,

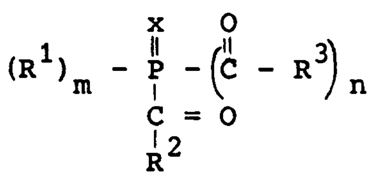

(I)

worin

$m = 1$, $n = 1$ und $x = O$

oder

$m = 2$, $n = 0$ und $x = O$ oder $S$

$R^1$ einen geradkettigen oder verzweigten $C_{1-6}$-Alkylrest,

einen Cyclohexyl-, Cyclopentyl-, Phenyl-, Naphthyl- oder Biphenylylrest,

einen Cyclopentyl-, Cyclohexyl-, Phenyl-, Naphthyl- oder Biphenylylrest, der substituiert ist durch F, Cl, Br, J, $C_1$-$C_{12}$-Alkyl und/oder $C_1$-$C_{11}$-Alkoxyl, einen S- oder N-haltigen 5- oder 6-gliedrigen heterocyclischen Ring;

$R^2$ und $R^3$, die gleich oder verschieden sind,

einen Cyclohexyl-, Cyclopentyl-, Phenyl-, Naphthyl- oder Biphenylylrest,

einen Cyclopentyl-, Cyclohexyl-, Phenyl-, Naphthyl- oder Biphenylylrest, der substituiert ist durch F, Cl, Br, J, $C_1$-$C_{12}$-Alkyl und/oder $C_1$-$C_{11}$-Alkoxyl, oder einen S- oder N-haltigen 5- oder 6-gliedrigen heterocyclischen Ring

bedeuten oder

$R^2$ und $R^3$ miteinander zu einem Ring verknüpft sind,

der 4 bis 10 Kohlenstoffatome enthält und durch 1 bis 6 $C_{1-4}$-Alkylreste substituiert sein kann.

2. Dentalmassen nach Anspruch 1, **dadurch gekennzeichnet**, daß sie zusätzlich
(d) 20 bis 79,99 Gew.%, bezogen auf die Summe aller polymerisierbarer Monomere, eines Acrylsäure- und/oder Methacrylsäureesters eines mindestens difunktionellen Alkohols enthalten.

3. Dentalmassen nach Anspruch 1 oder 2, **dadurch gekennzeichnet**, daß sie mindestens 20 Gew.%, bezogen auf die Summe aller polymerisierbarer Monomere, der Komponente (a) enthalten.

4. Dentalmassen nach einem der Ansprüche 1 bis 3, **dadurch gekennzeichnet**, daß mindestens ein Drittel der Poly-thiol-Verbindungen der Komponente (a) wenigstens trifunktionell ist.

5. Dentalmassen nach einem der Ansprüche 1 bis 4, **dadurch gekennzeichnet**, daß sie mindestens 20 Gew.%, bezogen auf die Summe aller polymerisierbarer Monomere, der Komponente (b) enthalten.

6. Dentalmassen nach einem der Ansprüche 1 bis 5, **dadurch gekennzeichnet**, daß mindestens ein Drittel der Poly-en-Verbindungen der Komponente (b) mindestens drei ethylenisch ungesättigte Gruppen enthält.

7. Dentalmassen nach einem der Ansprüche 1 bis 6, **dadurch gekennzeichnet**, daß die ethylenisch ungesättigten Gruppen der Poly-en-Verbindung(en) der Komponente (b) substituiert sind.

8. Dentalmassen nach einem der Ansprüche 1 bis 7, **dadurch gekennzeichnet**, daß die ethylenisch ungesättigten Gruppen der Poly-en-Verbindung(en) der Komponente (b) ausgewählt sind aus der Gruppe der O-Allyl-, N-Allyl-, O-Vinyl-, N-Vinyl- und p-Vinylphenolether-Gruppen.

9. Dentalmassen nach einem der Ansprüche 1 bis 8, **dadurch gekennzeichnet**, daß das Verhältnis (V) zwischen den ethylenisch ungesättigten Gruppen der Verbindungen der Komponente (b) und den Thiol-Gruppen der Verbindungen der Komponente (a) 0,2:1 bis 5:1 beträgt.

10. Dentalmassen nach Anspruch 9, **dadurch gekennzeichnet**, daß das Verhältnis (V) 0,75:1 bis 1,5:1 beträgt.

11. Dentalmassen nach einem der Ansprüche 1 bis 10, **dadurch gekennzeichnet**, daß die Poly-thiol-Verbindung(en) der Komponente (a) und/oder die Poly-en-Verbindung(en) der Komponente (b) ein Polyethermittelstück in ihrem Molekül aufweisen.

12. Dentalmassen nach einem der Ansprüche 1 bis 11, **dadurch gekennzeichnet**, daß sie 0,1 bis 3 Gew.%, bezogen auf die Summe aller polymerisierbarer Monomere, der Komponente (c) enthalten.

13. Dentalmassen nach einem der Ansprüche 1 bis 12, **dadurch gekennzeichnet**, daß sie als Photoinitiator 2,4,6-Trimethylbenzoyl-diphenylphosphinoxid oder Bis-(2,6-dichlorbenzoyl)-4′-n-propylphenylphosphinoxid enthalten.

14. Dentalmassen nach einem der Ansprüche 1 bis 13, **dadurch gekennzeichnet**, daß sie als übliche Hilfs- und Zusatzstoffe feinteilige Füllstoffe in einer Menge von bis zu 90 Gew.%, bezogen auf die Gesamtmasse, enthalten.

15. Dentalmassen nach Anspruch 14, **dadurch gekennzeichnet**, daß sie als Füllstoff silanisierte pyrogene Kieselsäure enthalten.

16. Verwendung der Dentalmassen gemäß einem der Ansprüche 1 bis 15 als dentale Abformmassen.

**Claims**

1. Photopolymerizable dental compositions which are curable with visible light and which apart from the usual auxiliary substances and additives contain polymerizable monomers of the group of the poly-thiol compounds each having at least two thiol groups and polymerizable monomers of the group of the poly-ene compounds each having at least two ethylenically unsaturated groups and at least one photoinitiator, characterized in that the compositions contain respectively related to the sum of all the polymerizable monomers

(a) at least 10 % by weight of one or more of the poly-thiol compounds,

(b) at least 10 % by weight of one or more of the poly-ene compounds and

(c) as photoinitiator 0.01 - 5 % by weight of at least one acyl phosphine compound of the general formula I

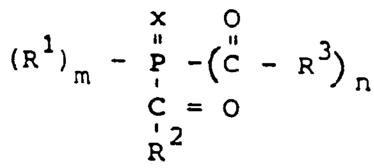

(I)

wherein

m = 1, n = 1 and x = 0

or

m = 2, n = 0 and x = O or S,

$R^1$ is a straight-chain or branched $C_{1-6}$-alkyl radical, a cyclohexyl, cyclopentyl, phenyl, naphthyl or biphenylyl radical,

a cyclopentyl, cyclohexyl, phenyl, naphthyl or biphenylyl radical which is substituted by F, Cl, Br, J, $C_1$-$C_{12}$-alkyl and/or $C_1$-$C_{11}$-alkoxyl, an S or N-containing 5 or 6-member heterocyclic ring;

$R^2$ and $R^3$, which are identical or different, denote

a cyclohexyl, cyclopentyl, phenyl, naphthyl or biphenylyl radical,

a cyclopentyl, cyclohexyl, phenyl, naphthyl or biphenylyl radical which is substituted by F, Cl, Br, J, $C_1$-$C_{12}$-alkyl and/or $C_1$-$C_{11}$-alkoxyl, or an S or N-containing 5 or 6-member heterocyclic ring

or

$R^2$ and $R^3$ are linked together to form a ring which contains 4 to 10 carbon atoms and can be substituted by 1 to 6 $C_{1-4}$-alkyl radicals.

**2.** Dental compositions according to claim 1, characterized in that they additionally contain

(d) 20 to 79.99 % by weight with respect to the sum of all the polymerizable monomers, of an acrylic acid ester and/or methacrylic acid ester of an at least difunctional alcohol.

**3.** Dental compositions according to claim 1 or 2, characterized in that they contain at least 20 % by weight of the component (a) with respect to the sum of all the polymerizable monomers.

**4.** Dental compositions according to any one of claims 1 to 3, characterized in that at least a third of the poly-thiol compounds of the component (a) is at least trifunctional.

**5.** Dental compositions according to any one of claims 1 to 4, characterized in that they contain at least 20 % by weight of the component (b) with respect to the sum of all the polymerizable monomers.

**6.** Dental compositions according to any one of claims 1 to 5, characterized in that at least a third of the poly-ene compounds of the component (b) contains at least three ethylenically unsaturated groups.

**7.** Dental compositions according to any one of claims 1 to 6, characterized in that the ethylenically unsaturated groups of the poly-ene compound(s) of the component (b) are substituted.

**8.** Dental compositions according to any one of claims 1 to 7, characterized in that the ethylenically unsaturated groups of the poly-ene compound(s) of the component (b) are selected from the group consisting of the O-allyl, N-allyl, O-vinyl, N-vinyl and p-vinyl phenol ether groups.

**9.** Dental compositions according to any one of claims 1 to 8, characterized in that the ratio (V) between the ethylenically unsaturated groups of the compounds of the component (b) and the thiol groups of the compounds of the component (a) is from 0.2:1 to 5:1.

**10.** Dental compositions according to claim 9, characterized in that the ratio (V) is from 0.75:1 to 1.5:1.

**11.** Dental compositions according to any one of claims 1 to 10, characterized in that the poly-thiol compound(s) of the component (a) and/or the poly-ene compound(s) of the component (b) have a polyether centre piece in their molecule.

**12.** Dental compositions according to any one of claims 1 to 11, characterized in that they contain from 0.1 to 3 % by weight of the component (c) with respect to the sum of all the polymerizable monomers.

**13.** Dental compositions according to any one of claims 1 to 12, characterized in that they contain as photoinitiator 2,4,6-trimethylbenzoyl-diphenyl phosphine oxide or bis-(2,6-dichlorobenzoyl)-4′-n-propylphenyl phosphine oxide.

**14.** Dental compositions according to any one of claims 1 to 13, characterized in that they contain as usual auxiliary substances and additives finely divided fillers in an amount of up to 90 % by weight with respect to the total composition.

**15.** Dental compositions according to claim 14, characterized in that they contain silanized pyrogenic silicic acid as a filler.

**16.** Use of the dental compositions according to any one of claims 1 to 15 as dental impression compositions.

## Revendications

**1.** Matériaux dentaires photopolymérisables, durcissables à la lumière visible, qui contiennent - outre des adjuvants et additifs habituels - des monomères polymérisables du groupe des composés de poly-thiol avec à chaque fois au moins deux groupes thiol et des monomères polymérisables du groupe des composés de poly-ène avec à chaque fois au moins deux groupes éthyléniquement insaturés ainsi qu'au moins un photoinducteur, caractérisés en ce que les matières contiennent, à chaque fois par rapport à la somme de tous les monomères polymérisables,

(a) au moins 10% en poids d'un ou plusieurs des composés de poly-thiol,
(b) au moins 10% en poids d'un ou plusieurs des composés de poly-ène et
(c) comme photoinducteur de 0,01 à 5% en poids d'au moins un composé d'acylphosphine de formule générale I

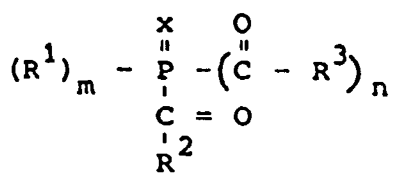

(I)

dans laquelle

m = 1, n = 1 et x = O

ou

m = 2, n = 0 et x = O ou S

$R^1$ représente un radical alkyle en $C_1$ à $C_6$ à chaîne droite ou ramifiée,

un radical cyclohexyle, cyclopentyle, phényle, naphtyle ou biphénylyle,

un radical cyclopentyle, cyclohexyle, phényle, naphtyle ou biphénylyle, qui est substitué par F, Cl, Br, I, alkyle en $C_1$ à $C_{12}$ et/ou alcoxyle en $C_1$ à $C_{11}$, un noyau hétérocyclique à 5 ou 6 chaînons contenant S ou N;

$R^2$ et $R^3$, qui sont identiques ou différents, représentent

un radical cyclohexyle, cyclopentyle, phényle, naphtyle ou biphénylyle,

un radical cyclopentyle, cyclohexyle, phényle, naphtyle ou biphénylyle, qui est substitué par F, Cl, Br, I, alkyle en $C_1$ à $C_{12}$ et/ou alcoxyle en $C_1$ à $C_{11}$, ou un radical hétérocyclique à 5 ou 6 chaînons contenant N ou S

ou

$R^2$ et $R^3$ sont reliés l'un à l'autre en un noyau qui contient de 4 à 10 atomes de carbone et qui peut être substitué par de un à six radicaux alkyle en $C_1$ à $C_4$.

**2.** Matériaux dentaires selon la revendication 1, caractérisés en ce qu'ils contiennent en outre
(d) de 20 à 79,99% en poids, par rapport à la somme de tous les monomères polymérisables, d'un ester de l'acide acrylique ou de l'acide méthacrylique d'un alcool au moins bifonctionnel.

**3.** Matériaux dentaires selon la revendication 1 ou 2, caractérisés en ce qu'ils contiennent au moins 20% en poids, par rapport à la somme de tous les monomères polymérisables, de composant (a).

**4.** Matériaux dentaires selon l'une des revendications 1 à 3, caractérisés en ce qu'au moins un tiers des composés de poly-thiol du composé (a) est au moins trifonctionnel.

**5.** Matériaux dentaires selon l'une des revendications 1 à 4, caractérisés en ce qu'ils contiennent au moins 20% en poids, par rapport à la somme de tous les monomères polymérisables, de composant (b).

**6.** Matériaux dentaires selon l'une des revendications 1 à 5, caractérisés en ce qu'au moins un tiers des composés de poly-ène du composant (b) contient au moins trois groupes éthyléniquement insaturés.

**7.** Matériaux dentaires selon l'une des revendications 1 à 6, caractérisés en ce que les groupes éthyléniquement insaturés du(des) composé(s) de poly-ène du composant (b) est(sont) substitué(s).

**8.** Matériaux dentaires selon l'une des revendications 1 à 7, caractérisés en ce que les groupes éthyléniquement insaturés du(des) composé(s) de poly-ène du composant (b) est(sont) choisi(s) dans l'ensemble des groupes O-allyl-, N-allyl, O-vinyl-, N-vinyl- et p-vinylphénoléther.

**9.** Matériaux dentaires selon l'une des revendications 1 à 8, caractérisés en ce que le rapport (V) entre les groupes éthyléniquement insaturés des composés du composant (b) et les groupe thiol des composés du composant (a) s'élève à 0,2:1 à 5:1.

**10.** Matériaux dentaires selon la revendication 9, caractérisés en ce que le rapport (V) s'élève à 0,75:1 à 1,5:1.

**11.** Matériaux dentaires selon l'une des revendications 1 à 10, caractérisés en ce le(s) composé(s) de polythiol du composant (a) et/ou le(s) composé(s) de poly-ène du composant (b) présentent une partie centrale polyéther dans leur molécule.

**12.** Matériaux dentaires selon une des revendications 1 à 11, caractérisés en ce qu'ils contiennent de 0,1 à 3% en poids, par rapport à la somme de tous les monomères polymérisables, de composant (c).

**13.** Matériaux dentaires selon l'une des revendications 1 à 12, caractérisés en ce qu'ils contiennent comme photoinducteurs le 2,4,6-triméthyl-benzoyl-diphényl-phosphinoxyde ou le bis-(2,6-dichlorobenzoyl)-4'-n-propylphénylphosphinoxyde.

**14.** Matériaux dentaires selon l'une des revendications 1 à 13, caractérisés en ce qu'ils contiennent comme additifs et adjuvants habituels des charges fines en une quantité allant jusqu'à 90% en poids, par rapport à la masse totale.

**15.** Matériaux dentaires selon la revendication 14, caractérisés en ce qu'ils contiennent comme charge de la silice pyrogénée silanisée

**16.** Application des matériaux dentaires selon l'une des revendications 1 à 15 comme matériaux de moulage.